(19) Europäisches Patentamt
European Patent Office
Office européen des brevets

(11) EP 1 378 239 A1

(12) **EUROPÄISCHE PATENTANMELDUNG**

(43) Veröffentlichungstag:
**07.01.2004 Patentblatt 2004/02**

(51) Int Cl.[7]: **A61K 33/26**, A61K 49/06, A61K 31/663

(21) Anmeldenummer: **03010660.3**

(22) Anmeldetag: **13.05.2003**

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**AL LT LV MK**

(30) Priorität: **22.05.2002 DE 10222481**

(71) Anmelder: **Eucro European Contract Research GmbH & Co. KG**
**41460 Neuss (DE)**

(72) Erfinder:
- **Greb, Wolfgang.**
  **D-40489 Düsseldorf (DE)**
- **Blum, Herbert.**
  **D-40595 Düsseldorf (DE)**
- **Roth, Marcel.**
  **D-49589 Düsseldorf (DE)**

(74) Vertreter: **Christophersen, Ruth et al**
**Christophersen & Partner,**
**Patentanwälte,**
**Feldstrasse 73**
**40479 Düsseldorf (DE)**

# (54) Kontrastmittel für die Verwendung in bildgebenden Verfahren

(57) Es wir die Verwendung von stabilisatorfreien superparamagnetischen Teilchen als Hilfsmittel in bildgebenden Verfahren sowie ein Kontrastmittel für die Verwendung in bildgebenden Verfahren, insbesondere zum Einsatz in der Kernspinresonanz-Spektroskopie bzw. -Tomographie und in der Röntgendarstellung enthaltend superparamagnetischen Teilchen beansprucht. Die superparamagnetischen Teilchen sind vorzugsweise ausgewählt aus Metalloxiden und/oder Metallen, insbesondere aus $\gamma$-$Fe_2O_3$, $Fe_3O_4$, $MnFe_2O_4$, $NiFe_2O_4$, $CoFe_2O_4$ und deren beliebigen Gemischen.

EP 1 378 239 A1

## Beschreibung

Die vorliegende Erfindung betrifft die Verwendung von superparamagnetischen Teilchen als Hilfsmittel in bildgebenden Verfahren sowie ein Kontrastmittel zum Einsatz in der medizinischen Diagnostik, insbesondere in bildgebenden Verfahren.

Bildgebende Verfahren, wie Röntgenuntersuchungen, MR-Tomographie, Szintigraphie usw. stellen in der medizinischen Diagnostik wichtige Hilfsmittel zur Darstellung von morphologischen und metabolischen Prozessen im Bereich des Skelettsystems bzw. krankhaften Veränderungen im Bereich der Weichteile dar. Bei der Anwendung dieser Verfahren ist in der Regel der Einsatz von sogenannten Kontrastmitteln notwendig. Diese bewirken bei den zu untersuchenden Körperteilen eine scharfe Abgrenzung zwischen gesundem Gewebe und krankhaften Veränderungen.

Als Kontrastmittel werden in der MR-Tomographie magnetische Teilchen eingesetzt, z.B. superparamagnetische Eisenoxidteilchen. Eine Übersicht über derartige Kontrastmittel wird in Eur.Radiol. (2001) 11, 2319-2331 gegeben. Der Anwendungsbereich der beschriebenen Kontrastmittel umfasst im Wesentlichen die Darstellung von inneren Organen, wie Leber, Milz, etc.

In der europäischen Patentschrift EP 0 689 430 B1 werden superparamagnetische Teilchen offenbart, die aus Eindomänenteilchen und Stabilisatorsubstanzen bestehen, wobei die Eindomänenteilchen aus Eisenoxid-, Eisenmischoxid- oder Eisen mit einer Teilchengröße im Bereich zwischen 3 und 20 nm zu stabilen, abbaubaren, ein definiertes Verhalten im Magnetfeld zeigenden Aggregaten zusammengelagert sind. Die Teilchengröße der Aggregate liegt im Bereich zwischen 10 und 1000 nm. Sie weisen auf ihrer Oberfläche eine monomolekulare Schicht auf, die aus Stabilisatorsubstanzen der Gruppe der phosphat-, diphosphat-, carboxylat-, polyphosphat-, thiophosphat-, phosphonat-, thiophosphonat-, sulfat-, sulfonat-, mercapto-, silantriol-, trialkoxysilangruppenhaltigen Polyalkylenglycole, der Kohlehydrate oder der phosphatgruppenhaltigen Nukleotide, deren Olygomere oder deren Polymere, welche weitere Bindungsstellen haben können. Wesentlich für die offenbarten superparamagnetischen Teilchen ist, dass diese zuerst aggregieren und anschließend die Stabilisatorsubstanzen an die Aggregatoberfläche gebunden werden, so dass sich die Eigenschaften der Aggregate ändern. Ein mögliches Einsatzgebiet der beschriebenen superparamagnetischen Teilchen ist die Verwendung als Kontrastmittel für die Kernspin-Diagnostik.

In der europäischen Patentanmeldung EP 0 284 549 A2 werden magnetische Flüssigkeitszusammensetzungen offenbart, die eine physiologisch verträgliche Dispersion aus fein verteilten superparamagnetischen Teilchen in Wasser und eine für die Stabilisierung und für die chemische Bindung von diagnostischen und pharmakologischen wirksamen Substanzen ausreichende Menge an reaktiven Stabilisatorsubstanzen enthalten. Die Stabilisatorsubstanzen sind mit der Oberfläche der superparamagnetischen Teilchen über Phosphat-, Phosphonatoder Carboxylatgruppen chemisch verbunden. Diese magnetischen Flüssigkeitszusammensetzungen werden u.a. in der NMR-Diagnostik eingesetzt.

Die Darstellung von Knochen etc. erfolgt in der Regel durch Röntgenuntersuchung oder Szintigraphie. Durch die Szintigraphie wird eine zweidimensionale, intensitätsproportionale Darstellung der selektiven Verteilung eines γ-Strahlers in einem lebenden Organ erhalten. Während die üblicherweise verwendeten Kontrastmittel mit Nachteilen und Nebenwirkungen wie Unverträglichkeit durch den Patienten verbunden sind, werden in der Szintigraphie als Kontrastmittel Radionukleotide eingesetzt, die die langfristigen Nebenwirkungen von radioaktiven Substanzen zeigen.

Die verwendeten Kontrastmittel werden oral, durch Infusionen oder Injektionen appliziert. Das applizierte Mittel verteilt sich im Blutkreislauf oder über den Blutkreislauf im Körper und reichert sich in Organen an. Für die Untersuchung des Gastrointestinaltraktes erfolgt die Applikation der Kontrastmittel in der Regel oral.

Eine gezielte Anreicherung der superparamagnetischen Teilchen, beispielsweise am Knochen, ist mit den aus dem Stand der Technik bekannten Mitteln nicht möglich.

Nur wenn diese Teilchen mit gewebebindenden spezifischen Substanzen als Targeting-Hilfe beladen sind, werden sie organspezifisch gebunden und sind dann zur Bildgebung besonders geeignet.

Für die Behandlung und Darstellung von Knochen, Knochenumbauprozessen und Läsionen, sowie Knochenmetastasen verschiedener Genese werden Bisphosphonate aufgrund ihrer besonderen Bindungsfähigkeit an Apatit, den anorganischen Hauptbestandteil von Knochengewebe eingesetzt. Stand der Technik ist hier z.B. die Technetium-99-Knochenszintigraphie mittels geeigneter Bisphosphonate, die überwiegend parenteral appliziert/injiziert werden. Zur Behandlung von Knochenerkrankungen werden sie auch oral verabreicht.

Das sich am Knochenapatit anreichernde Bisphosphonat fixiert demnach am Knochen die daran gebundenen Atome oder Moleküle (drug targeting), die ihrerseits lokal ihre Wirkung entfalten können. Im Falle der Kontrastmittelanwendung werden die Eisenpartikel an den Knochen/Skelett transportiert und ermöglichen dort die bildliche Darstellung der Knochenstruktur bzw. der Höhlen des Knochenmarks (negative Darstellung). Je nach eingesetztem Bisphosphonat variiert dessen Anreicherung am Apatit des gesunden Knochens, der Knochenumbauzonen (wie z.B. Wachstumsfragen, rheumatische Entzündungsprozesse, Knochenabbau oder Knochentumorgeschehen). Entsprechend kann das Kontrastmittel selektiert werden.

Der vorliegenden Erfindung lag demgemäß die Aufgabe zugrunde, ein Hilfsmittel für die Verwendung in bildgebenden Verfahren zur Verfügung zu stellen, das gezielt in dem zu untersuchenden Organ oder Körperteil angereichert werden kann, wobei die Nachteile bzw. Nebenwirkungen einer Strahlenbelastung, wie durch radioaktiven Substanzen oder auch Röntgenstrahlen, reduziert werden und falls möglich ganz verzichtet werden soll.

Gegenstand der vorliegenden Erfindung ist demgemäß die Verwendung von stabilisatorfreien superparamagnetischen Teilchen als Hilfsmittel in bildgebenden Verfahren.

Hilfsmittel im Sinne der vorliegenden Erfindung bedeutet, daß die erfindungsgemäßen superparamagnetischen Teilchen als Kontrastmittel oder zur Sensibilisierung der zu messenden physikalischen Größen, wie der Kernspinresonanz, eingesetzt werden. Beispielsweise können die superparamagnetischen Teilchen in Röntgenverfahren als Kontrastmittel eingesetzt werden, dabei macht man sich den Effekt zunutze, daß die superparamagnetischen Teilchen die Röntgenstrahlen schwächer oder stärker aborbieren als das benachbarte Körpergeweben/Knochen, wodurch die Darstellung der Körperstrukturen als Bild möglich ist. Bei Kernspinresonanzverfahren können die erfindungsgemäßen superparamagnetischen Teilchen eine Kontrastverstärkung bewirken, indem sie die Wasserstoffbindungen in ihrer Umgebung anregen/sensibilisieren, was in Kernspinresonanzverfahren zu einem stärkeren Meßsignal führt.

Ein weiterer Gegenstand ist ein Kontrastmittel bzw. Kontrasthilfsmittel für die Verwendung in bildgebenden Verfahren, enthaltend stabilisatorfreie superparamagnetische Teilchen.

Das Kontrastmittel bzw. Kontrasthilfmittel (im folgenden wird nur der Ausdruck Kontrastmittel verwendet) wird vorzugsweise in der Kernspinresonanz-Spektroskopie bzw. -Tomographie und in der Röntgendarstellung eingesetzt.

Das erfindungsgemäß eingesetzten superparamagnetischen Teilchen dienen in bildgebenden Verfahren als Hilfsmittel, vorzugsweise werden als Kontrastmittel bzw. zur Kontrastverstärkung in der Kernspinresonanz-Spektroskopie bzw. -Tomographie und in der Röntgendarstellung verwendet.

In einer möglichen Ausführungsform der vorliegenden Erfindung werden neben den superparamagnetischen Teilchen zusätzlich biologisch und pharmakologische Wirkstoffe, wie chemische Substanzen, Proteine einschließlich Antikörper, Peptide, Oligonucelotide verwendet. In dieser Ausführungsform werden die Wirkstoffe gemeinsam mit den superparamagnetischen Teilchen gezielt an den Wirkort transportiert. An ihrem Wirkort reagieren die Aktivstoffe speziellen biologischen Wirkstoffen, wie Rezeptoren, Zellen (Makrophagen), Geweben (z. B. Milz) usw. Sie binden aktiv an derartaige Proteine oder werden aktiv von Zellen oder Geweben aufgenommen. Gleiches gilt auch für Aktivstoffderivate, Konglomerate oder Transportsysteme mit entsprechender Aktivgruppe. Gemäß dieser Ausführungsform transportiert und bindet der Aktivstoff die superparamagnetischen Teilchen an bestimmte Strukturen im Körper, wo es selbst als Kontrastmittel in bildgebenden Verfahren oder als Hilfsmittel in diesen Verfahren auf lokaler Ebene fungiert und die gewünschten Strukturen "sichtbar" darstellen kann.

Die erfindungsgemäß enthaltenen superparamagnetischen Teilchen werden vorzugsweise aus den für den jeweiligen Anwendungszweck geeigneten superparamagnetischen Substanzen wie Metalloxiden und/oder Metallen ausgewählt, wobei wegen ihrer physiologischen Verträglichkeit für den in-vivo-Einsatz besonders bevorzugt $\gamma$-$Fe_2O_3$, $Fe_3O_4$, $MnFe_2O_4$, $NiFe_2O_4$, $CoFe_2O_4$ und deren beliebigen Gemische eingesetzt werden. $Fe_3O_4$ (Magnetit) ist ganz besonders bevorzugt. Als geeignete Metalle können Fe, Co, Ni sowie deren Legierungen ggf. auch mit anderen Metallen genannt werden.

Die superparamagnetischen Teilchen können ohne die im Stand der Technik beschriebenen Stabilisatoren, d.h. stabilisatorfrei, eingesetzt werden. Stabilisatorfrei bedeutet in der vorliegenden Erfindung, dass die magnetischen Partikel ohne Zusatz von Hilfsmitteln, wie Emulgatoren oder Oberflächenbeschichtungen, wie sie im Stand der Technik beschrieben werden, mit Aktivstoffen beaufschlagt sind bzw. von diesen umhüllt sind. Auch ist eine Behandlung von mit Aktivstoffen beladenen Partikeln nicht erforderlich und vorzugsweise ausgeschlossen. In der einfachsten Ausgestaltung der vorliegenden Erfindung besteht das erfindungsgemäße System aus magnetischem Partikel und Aktivstoff.

Werden die superparamagnetischen Teilchen in Kombition mit anderen Stoffen eingesetzt, so befinden sich diese üblicherweise teilweise auf der Oberfläche der magnetischen Partikel. Das bedeutet, dass die weitere Stoffe unmittelbar auf der Oberfläche der Partikel aufgebracht sind. Die Partikel können auch von den weiteren Stoffen umhüllt sein, was zum Beispiel.dann der Fall ist, wenn die weiteren Stoffe aufgrund ihrer Struktur, Form oder Größe die magnetischen Partikel umgeben aber nicht auf der Oberfläche direkt aufgebracht sind. Eine Umhüllung liegt beispielsweise dann vor, wenn als weiterer Stoffe Zellen, Zellkulturen bzw. Zellbestandteile verwendet werden und die magnetische Partikel im inneren der Zellen, Zellkulturen bzw. Zellbestandteile vorliegen, oder wenn die weiteren Stoffe aufgrund ihrer Molekülgröße die Struktur eines Knäuels aufweisen, in dessen Inneren sich die magnetischen Partikel befinden.

Die erfindungsgemäß eingesetzten superparamagnetischen Teilchen weisen in der Regel vorzugsweise eine Teilchengröße von 1 bis 500 nm auf, vorzugsweise von 1 - 50, insbesondere von 10 - 20 nm auf, wobei hier die einzelnen diskreten Kristallite gemeint sind. Es können auch Agglomerate vorliegen, deren Gesamteilchengröße unterhalb von 5 µm, insbesondere oberhalb von 50 nm und unterhalb von 1000 nm liegt.

Die voranstehend genannte Teilchengröße ist für beliebige Applikationsformen geeignet. Es hat sich jedoch für die orale Verabreichung und für langsame Infusionen als vorteilhaft erwiesen, wenn die Teilchengröße der superparamagnetischen Teilchen zwischen 50 und 500 nm, insbesondere zwischen 200 und 500 nm liegt. Oral verabreichte

Kontrastmittel mit einer Teilchengröße in diesem Bereich sind insbesondere für die Darstellung des Gastrointestinaltraktes und anderen Hohlorganen, wie z.B. Blase, Vagina, Nasennebenhöhlen oder Zysten sowie Blutgefäße und offene Wunden geeignet.

Soll das erfindungsgemäße Kontrastmittel durch Infusion oder Injektion verabreicht werden, haben sich Teilchengrößen der superparamagnetischen Teilchen zwischen 1 und 200 nm, insbesondere zwischen 1 und 50 nm, als besonders geeignet erwiesen.

Zur Darstellung von Knochen bzw. Veränderungen am Knochen werden vorzugsweise superparamagnetische Teilchen eingesetzt, in denen die Teilchengröße der superparamagnetischen Teilchen zwischen 10 und 20 nm liegt.

Die volumengewichtete mittlere Kristallitgröße ist mit Röntgenbeugungsverfahren, insbesondere über eine Scherrer-Analyse, bestimmbar. Das Verfahren ist beispielsweise beschrieben in: C. E. Krill, R. Birringer: "Measuring average grain sizes in nanocrystalline materials", Phil. Mag. A 77, S. 621. (1998). Demnach kann die volumengewichtete mittlere Kristallitgröße D bestimmt werden durch den Zusammenhang

$$D = K\lambda/\beta\cos\theta.$$

Dabei ist $\lambda$ die Wellenlänge der verwendeten Röntgenstrahlung, $\beta$ ist die volle Breite auf halber Höhe des Reflexes an der Beugungsposition $2\theta$. K ist eine Konstante der Größenordnung 1, deren genauer Wert von der Kristallform abhängt. Man kann diese Unbestimmtheit von K vermeiden, indem man die Linienverbreiterung als integrale Weite $\beta_i$ bestimmt, wobei $\beta_i$ definiert ist als die Fläche unter dem Röntgenbeugungsreflex geteilt durch dessen maximaler Intensität $I_0$:

$$\beta_i = 1/I_0 \int_{2\theta_1}^{2\theta_2} I(2\theta)d(2\theta)$$

Dabei sind die Größen $2\theta_1$ und $2\theta_2$ die minimale und maximale Winkelposition des Bragg-Reflexes auf der 2θ-Achse. $I(2\theta)$ ist die gemessene Intensität des Reflexes als Funktion von $2\theta$. Unter Verwendung von diesem Zusammenhang ergibt sich als Gleichung zur Bestimmung der volumengewichteten mittleren Kristallitgröße D: $D = \lambda/\beta_i\cos\theta$.

Es wurde festgestellt, dass die erfindungsgemäß als gewebespezifische Substanz enthaltenen Diphosphonsäuren und deren Salze durch die in den Molekülen vorhandenen aktiven Molekülgruppen die Fähigkeit besitzen, sich gezielt an Strukturgewebe zu binden, wodurch eine besonders gute Anreicherung der Kontrastmittel im zu untersuchenden Organ erfolgt.

In eine bevorzugten Ausführungsform werden die superparamagnetischen Teilchen in Kombination mit einer gewebespezifischen Substanz ausgewählt aus Diphosphonsäuren und deren physiologisch unbedenklichen Salzen eingesetzt. Die Diphosphonsäuren und deren physiologisch unbedenklichen Salze zeichnen sich durch ihre guten Bindungseigenschaften an körpereigene Gewebe, insbesondere, kalkhaltige Gewebe (Verkalkungen) und gegenüber Knochen aus. Neben dem Knochen/Skelett als positivem Target werden auch die entsprechenden Hohlräume dargestellt, z.B. das Knochenmark. Die Kombination aus superparamagnetischen Teilchen und Diphosphonsäure oder deren physiologisch unbedenklichen Salzen ist besonders vorteilhaft bei der Darstellung von Knochen, d.h. Skelett, und insbesondere von Feinstrukturen oder Umbauzonen wie Metastasen. Die Diphosphonsäure oder deren physiologisch unbedenklichen Salze reichern sich in den Umbauzonen, wie Metastasen, an, wodurch bei einer Kombination aus superparamagnetischen Teilchen und Diphosphonsäure(salz) auch die superparamagnetischen Teilchen angereichtert werden und bei der Darstellung im Röntgenbild die Strukturänderung wiedergeben können. Wegen der schlechten Darstellbarkeit der Feinstrukturen und Umbauzonen erfolgt derzeit bei Verdacht auf Metastasen im Knochengerüst regelmäßig eine radioaktive Szintigraphie unter Einsatz von Technetium-Verbindungen.

Als besonders geeignete Diphosphonsäuren haben sich geminale Diphosphonsäuren bzw. geminale Biphosphonocarbonsäuren und/deren physiologisch verträglichen Salze mit der allgemeinen Formel I erwiesen,

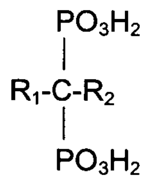

I

in der ist

$R_1$ COOH, ein linearer oder verzweigter Alkylrest mit 1 bis 10 Kohlenstoffatomen, der gegebenenfalls substituiert sein kann durch Substituenten wie Aminogruppen, N-Mono- bzw. N-Dialkylaminogruppen, wobei die Alkylgruppen 1 bis 5 C-Atome und/oder SH-Gruppen enthalten können, oder ein substituierter oder unsubstituierter carbo- oder heterocyclischer Aryl/Cycloalkanrest, der auch ein kondensiertes Ringsystem mit bis zu drei Ringen bilden kann, der ggf. ein oder mehrere Heteroatome , besonders bevorzugt sind N-Atome als Heteroatome, und als Substituenten verzweigte und unverzweigte Alkylreste mit 1 bis 6 C-Atomen, freie oder mono- resp. dialkylierte Aminogruppen mit 1 bis 6 C-Atomen oder Halogenatome aufweisen kann, und

$R_2$ gleich OH, COOH, ein Halogenatom, vorzugsweise Cl, H oder $NH_2$.

Als Beispiele für geeignete Salze der Verbindungen mit der Formel I können Alkalimetallsalze, Erdalkalimetallsalze, Ammoniumsalze und/oder Ethanolaminsalze genannt werden.

Derartige Verbindungen eignen sich insbesondere für die Behandlung von osteoporotischen Erkrankungen, wobei folgende Verbindungen besonders bevorzugt sind:

1-Methyl-1-hydroxy-1,1-diphosphonsäure (MDP),
1,1-Diphosphono-propan-2,3-dicarbonsäure (DPD),
3-(Methyl-pentylamino)-1-hydroxypropan-1,1-diphosphonsäure (Ibandronsäure),
1-Hydroxyethan-1,1-diphosphonsäure (Etidronsäure;HEDP),
Dichlormethandiphosphonsäure (Clodronsäure),
3-Amino-1-hydroxypropan-1,1-diphosphonsäure (Pamidronsäure),
4-Amino-1-hydroxybutan-1,1-diphosphonsäure (Alendronsäure),
2-(3-Pyridin)-1-hydroxyethan-1,1-diphosphonsäure (Risedronsäure),
4-Chlorphenylthiomethan-1,1-di-phosphonsäure (Tiludronsäure),
Pyrimidinyl-1-hydroxyethan-1,1-diphosphonsäure (Zoledronsäure),
Cycloheptylaminomethan-1,1-diphosphonsäure (Cimadronsäure),
6-Amino-1-hydroxyhexan-1,1-diphosphonsäure (Neridronsäure),
3-(N,N-Dimethylamino)-1-hydroxypropan-1,1-diphosphonsäure (Olpadronsäure),
3-Pyrrol-1-hydroxypropan-1,1-diphosphonsäure und/oder
2-Pyrimidazol-1-hydroxyethan-1,1-diphosphonsäure (Minodronsäure)
Azacycloheptan-2,2-diphosphonsäure
sowie deren physiologisch verträglichen Salzen.

Die Diphosphonsäuren als gewebespezifische Substanz können auf beliebige Weise auf die superparamagnetischen Teilchen aufgebracht oder an diese gebunden sein. Üblicherweise befinden sich die Diphosphonsäuren auf der Oberfläche der superparamagnetischen Teilchen und/oder, wenn diese als Agglomerate vorliegen, auch in deren Hohlräumen. Die Diphosphonsäuren könne auch über kovalente und/oder ionische Bindungen, ggf. über Spacergruppen, oder über Van-der-Waal'sche-Wechselwirkungen an die Teilchen gebunden sein.

Das erfindungsgemäße Kontrastmittel enthält sowohl superparamagnetische Teilchen als auch eine gewebespezifische Substanz, wobei die gewebespezifische Substanz vorzugsweise auf den superparamagnetischen Teilchen adsorbiert ist. In einer möglichen Ausführungsform können die superparamagnetischen Teilchen und die gewebespezifische Substanz als Feststoffe mit einander vermischt sein. Es hat sich jedoch als besonders vorteilhaft erwiesen, wenn die gewebespezifische Substanz bereits bei der Bildung der magnetischen Teilchen vorliegt, z.B. wenn die superparamagnetischen Teilchen durch eine größenkontrollierte Fällung im wässerigen Medium mittels alkalischer Substanzen oder durch Reduktion von Metallkationen hergestellt werden. Durch die in situ erzeugte große Partikeloberfläche kann eine optimale Adsorption der gewebespezifischen Substanz an die Oberfläche der Teilchen mittels reaktiver Gruppen, wie OH-, SH-, Hydroxid-, Amino-, Carboxyl-, Ether-, Sulfo-, Phosphonsäuregruppen usw. erfolgen. Es ist auch möglich, die gewebespezifische Substanz nachträglich auf die gefällten superparamagnetischen Teilchen aufzubringen, z.B. durch Suspension der ungecoateten (nicht modifizierten) superparamagnetischen Partikel in einer die gewebespezifische Substanz bzw. ein Substanzgemisch enthaltenden flüssigen Phase, vorzugsweise Wasser.

In einer möglichen Ausführungsform der vorliegenden Erfindung können die Aktivstoffe auch über sog. Spacer-Gruppen an die magnetischen Partikel gebunden werden. Spacer sind kurze organische Molekül-Ketten, die bei der Immobilisierung von Molekülen auf Trägern genutzt werden, wobei die Spacer-Moleküle keine Beschichtung darstellen. Spacer können beispielsweise eingesetzt werden, wenn die Aktivstoffe keine polaren Gruppen oder ionischen Gruppen aufweisen. Die Spacer-Moleküle können die Bindung zwischen magnetischen Partikeln und den Aktivstoffen verbessern. Sie weisen vorzugsweise eine oder mehrere polare Gruppe(n) auf. Als Beispiele kann auf die bereits genannten Gruppen verwiesen werden. Insbesondere wenn kationische Aktivstoffe verwendet werden, haben sich Spacer mit zwei polaren Gruppen, wie Aminocarbonsäuren, Diamine, Betaine, Dicarbonsäuren, Aminophosphonate, etc. als geeignet erwiesen.

In einer weiteren möglichen Ausführungsform werden sogenannte Agglomerate von magnetischen Partikeln eingesetzt, die aus Agglomeraten von Nanopartikeln, d.h. von Kristalliten mit einer Teilchengröße unter 100 nm, bestehen. Diese Agglomerate können aus einzelnen Kristalliten bestehen, welche an ihrer Kontaktfläche entweder reversibel agglomeriert oder irreversibel durch Kovaleszens, d.h. durch Zusammenwachsen über die Korngrenzen hinweg, agglomeriert sind. Ein Vorteil von Agglomeraten besteht darin, dass sie sowohl eine äußere als auch eine innere Oberfläche, d.h. Hohlräume aufweisen, so dass die gewebespezifische Substanz innen und außen gebunden werden können. Agglomerate können beispielsweise erhalten werden, indem die magnetischen Partikel in Abwesenheit eines Wirkstoffs gefällt werden, durch Trocknung oder Gefriertrocknung wirkstofffreier oder mit gewebespezifischer Substanz beladenen Teilchen mit anschließender Redispergierung, Agglomeratbildung, welche durch die Synthesebedingungen gesteuert werden kann, wie Temperaturerhöhung, Einstellung des pH-Wertes, hoher Elektrolytgehalt oder durch eine geeignete Nachbehandlung der gefällten Partikel bei Temperaturen von über 100°C.

Die erfindungsgemäßen Mittel werden als übliche pharmazeutische Applikationsformen verwendet werden, wie parenteral, iv, Inhalation, oral, Instillation in Körperhöhlen, auch intraoperativ.

Das erfindungsgemäße Kontrastmittel kann, wie bereits erwähnt, oral, durch Infusion oder Injektion verabreicht werden. Für diese Verabreichungsformen wird das erfindungsgemäße Kontrastmittel vorzugsweise in eine geeignete pharmazeutische Zubereitung überführt, wobei insbesondere Suspensionen, Emulsionen und liposomale Systeme zu nennen sind. Für die orale Verabreichung können zusätzlich Tabletten oder Kapseln genannt werden.

Das erfindungsgemäße Kontrastmittel wird zur besseren Darstellung von zu untersuchenden Organen und Körperteilen in der medizinischen Diagnostik eingesetzt. Besonders geeignete bildgebende Verfahren sind die MR-Tomographie, und andere Kernspinverfahren zur makround mikroskopischen Darstellung.

Ein weiterer Gegenstand der vorliegenden Erfindung ist demgemäß die Verwendung des voranstehend beschriebenen Kontrastmittels in der medizinischen Diagnostik, insbesondere in der MR-Tomographie zur Darstellung von Knochenskelett und Läsionen am Knochen.

## Beispiele

### Herstellungsbeispiele

1. 6.48g $FeCl_3$ wurden in 40 g entionisiertem Wasser gelöst. Außerdem wurden 3,97 g $FeCl_2*4H_2O$ in einer Mischung aus 8 ml entionisiertem Wasser und 2 ml 37%iger Salzsäure gelöst. Kurz vor Einsatz der Lösungen im Fällungsprozess wurden die beiden Mischungen vereinigt.

2. In einem Becherglas wurden 400 ml entionisiertes Wasser mit 10 g NaOH und 0,2 g 1-Methyl-1-hydroxy-1,1-diphosphonsäure (MDP) verrührt. Nach Abkühlen wurden hierzu unter starkem Rühren die salzsaure Eisensalzlösung aus 1 gegossen. Mittels Magnetfeld wurde der gebildete schwarze Niederschlag sedimentiert und die überstehende Lösung abdekantiert. Anschließend wurde das gefällte Material mehrmals in Wasser aufgenommen und dekantiert, um Fremdionen zu entfernen. Anschließend wurden 0,5 g MDP und 100 ml Wasser zugegeben. Nach 1stündigem Rühren bei 40 °C wurde 12 h lang bei RT nachgerührt. Nicht suspendierte Anteile wurden durch Zentrifugieren (5000-11000 Umdrehungen/Minute) abgetrennt. Auf diese Weise wurde eine magnetische Flüssigkeit erhalten, die im Rotationsverdampfer bis zum Erhalt des gewünschten Feststoffgehalts eingeengt wurde.

3. Beispiel 2 wurde wiederholt, wobei anstelle von 1-Methyl-1-hydroxy-1,1-diphosphonsäure 1,1-Diphosphonopropan-2,3-dicarbonsäure (DPD) verwendet wurde.

**Patentansprüche**

1. Verwendung von stabilisatorfreien superparamagnetischen Teilchen als Hilfsmittel in bildgebenden Verfahren.

2. Verwendung nach Anspruch 1, **dadurch gekennzeichnet, dass** die superparamagnetischen Teilchen zur Kontrastverstärkung in der Kernspinresonanz-Spektroskopie bzw. -Tomographie und in der Röntgendarstellung verwendet werden.

3. Verwendung nach Anspruch 1 oder Anspruch 2, **dadurch gekennzeichnet, dass** zusätzlich biologisch und pharmakologische Wirkstoffe, wie chemische Substanzen, Proteine einschließlich Antikörper, Peptide, Oligonucelotide verwendet werden.

4. Verwendung nach einem der Ansprüche 1 bis 3, **dadurch gekennzeichnet, dass** die superparamagnetischen Teilchen ausgewählt sind aus Metalloxiden und/oder Metallen, insbesondere aus $\gamma$-$Fe_2O_3$, $Fe_3O_4$, $MnFe_2O_4$, $NiFe_2O_4$, $CoFe_2O_4$ und deren beliebigen Gemischen.

5. Verwendung nach einem der Ansprüche 1 bis 4 **dadurch gekennzeichnet, dass** die superparamagnetischen Teilchen eine Teilchengröße von 1 bis 500 nm, vorzugsweise von 1 - 50, insbesondere von 10 - 20 nm, aufweisen.

6. Verwendung nach einem der Ansprüche 1 bis 5 als Kontrastmittel für die Verwendung in bildgebenden Verfahren.

7. Verwendung nach Anspruch 6, **dadurch gekennzeichnet, dass** die superparamagnetische Teilchen in Kombination mit einer gewebespezifischen Substanz ausgewählt aus Diphosphonsäuren und deren physiologisch unbedenklichen Salzen eingesetzt wirden.

8. Verwendung nach Anspruch 7, **dadurch gekennzeichnet, dass** die Bisphosphonsäuren und deren Salze ausgewählt sind aus geminalen Bisphosphonsäuren und/oder deren physiologisch verträglichen Salzen der allgemeinen Formel I

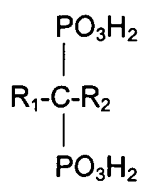

I

in der ist
$R_1$ COOH, ein linearer oder verzweigter Alkylrest mit 1 bis 10 Kohlenstoffatomen, der gegebenenfalls substituiert sein kann durch Substituenten wie Aminogruppen, N-Mono- bzw. N-Dialkylaminogruppen, wobei die Alkylgruppen 1 bis 5 C-Atome und/oder SH-Gruppen enthalten können, oder ein substituierter oder unsubstituierter carbo- oder heterocyclischer Aryl-/Cycloalkanrest, der auch ein kondensiertes Ringsystem mit bis zu drei Ringen bilden kann, der ggf. ein oder mehrere Heteroatome , besonders bevorzugt sind N-Atome als Heteroatome, und als Substituenten verzweigte und unverzweigte Alkylreste mit 1 bis 6 C-Atomen, freie oder mono- resp. dialkylierte Aminogruppen mit 1 bis 6 C-Atomen oder Halogenatome aufweisen kann, und
$R_2$ gleich OH, COOH, ein Halogenatom, vorzugsweise Cl, H oder $NH_2$.

9. Verwendung nach Anspruch 8, **dadurch gekennzeichnet, dass** die Bisphosphonsäure und deren physiologisch verträglichen Salze ausgewählt sind aus 1-Methyl-1-hydroxy-1,1-diphosphonsäure (MDP), 1,1-Diphosphono-propan-2,3-dicarbonsäure (HDP), 3-(Methyl-pentylamino)-1-hydroxypropan-1,1-diphosphonsäure (Ibandronsäure), 1-Hydroxyethan-1,1-diphosphonsäure (Etidronsäure), Dichlormethandiphosphonsäure (Clodronsäure), 3-Amino-1-hydroxypropan-1,1-diphosphonsäure (Pamidronsäure), 4-Amino-1-hydroxybutan-1,1-diphosphonsäure (Alendronsäure), 2-(3-Pyridin)-1-hydroxyethan-1,1-diphosphonsäure (Risedronsäure), 4-Chlorphenylthiomethan-1,1-diphosphonsäure (Tiludronsäure), Pyrimidinyl-1-hydroxyethan-1,1-diphosphonsäure (Zoledronsäure), Cycloheptylaminomethan-1,1-diphosphonsäure (Cimadronsäure), 6-Amino-1-hydroxyhexan-1,1-diphosphonsäure (Neridronsäure), 3-(N,N-Dimethylamino)-1-hydroxypropan-1,1-diphosphonsäure (Olpadronsäure), 3-Pyrrol-1-hydroxypropan-1,1-diphosphonsäure und/oder 2-Pyrimidazol-1-hydroxyethan-1,1-diphosphonsäure (Minodronsäure).

**10.** Verwendung nach einem der Ansprüche 1 bis 9, **dadurch gekennzeichnet, dass** die verwendeten Substanzen parenteral, iv, Inhalation, oral, per Instillation in Körperhöhlen oder intraoperativ appliziert werden.

**11.** Verwendung nach einem der Ansprüche 1 bis 10, **dadurch gekennzeichnet, dass** die Substanzen als Suspension, Emulsion und/oder liposomale System vorliegen.

**12.** Verwendung nach einem der Ansprüche 1 bis 11 in der medizinischen Diagnostik.

**13.** Verwendung nach einem der Ansprüche 1 bis 11 zur Darstellung vom Knochenskelett und Läsionen am Knochen.

**14.** Kontrastmittel für die Verwendung in bildgebenden Verfahren, insbesondere zum Einsatz in der Kernspinresonanz-Spektroskopie bzw. -Tomographie und in der Röntgendarstellung enthaltend superparamagnetischen Teilchen, vorzugsweise ausgewählt aus Metalloxiden und/oder Metallen, insbesondere aus $\gamma$-$Fe_2O_3$, $Fe_3O_4$, $MnFe_2O_4$, $NiFe_2O_4$, $CoFe_2O_4$ und deren beliebigen Gemischen.

Europäisches Patentamt

# EUROPÄISCHER RECHERCHENBERICHT

Nummer der Anmeldung
EP 03 01 0660

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 6 123 920 A (NAEVESTAD ANNE ET AL)<br>26. September 2000 (2000-09-26)<br>* Spalte 14, Zeile 56-61; Ansprüche 10,11,13,15; Beispiel 6 *<br>* Spalte 12, Zeile 37-39 *<br>--- | 1-6,<br>10-14 | A61K33/26<br>A61K49/06<br>A61K31/663 |
| X,D | EP 0 689 430 A (SILICA GEL GMBH ;PILGRIMM HERBERT (DE)) 3. Januar 1996 (1996-01-03)<br>* Seite 3, Zeile 47 - Seite 4, Zeile 4; Beispiele 1,2 * | 1-6,<br>10-12,14 | |
| Y | * Seite 9, Zeile 14-37 *<br>--- | 7-9,13 | |
| X | WO 90 01899 A (ADVANCED MAGNETICS INC)<br>8. März 1990 (1990-03-08)<br>* Ansprüche 1,2,5,11 *<br>--- | 1-6,<br>10-12,14 | |
| X | WO 01 89584 A (KLAVENESS JO ;NYCOMED IMAGING AS (NO); TOLLESHAUG HELGE (NO))<br>29. November 2001 (2001-11-29)<br>* Seite 17, Absatz 7 - Seite 18, Absatz 1 *<br>* Seite 49, Absatz 4 - Seite 50, Absatz 1 * | 1-6,<br>10-14 | RECHERCHIERTE SACHGEBIETE (Int.Cl.7)<br>A61K |
| Y | * Seite 23, Zeile 4 *<br>--- | 7-9,13 | |
| X | EP 0 988 864 A (LUBOLDT WOLFGANG DR MED DIPL P) 29. März 2000 (2000-03-29)<br>* Spalte 2, Absatz 9; Ansprüche 1,6 *<br>--- | 1-6,10,<br>12,14 | |
| X | US 4 770 183 A (JOSEPHSON LEE ET AL)<br>13. September 1988 (1988-09-13)<br>* Ansprüche 1-3,9,10 *<br>--- | 1-6,10,<br>12,14 | |
| X | WO 02 22011 A (DIAGNOSTIKFORSCHUNG INST)<br>21. März 2002 (2002-03-21)<br>* Ansprüche 1-8 *<br>---<br>-/-- | 1-6,<br>10-12,14 | |

Der vorliegende Recherchenbericht wurde für alle Patentansprüche erstellt

| Recherchenort | Abschlußdatum der Recherche | Prüfer |
|---|---|---|
| MÜNCHEN | 29. August 2003 | Ansaldo, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

Europäisches Patentamt

# EUROPÄISCHER RECHERCHENBERICHT

Nummer der Anmeldung
EP 03 01 0660

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | US 5 323 780 A (LIEBIG THOMAS ET AL) 28. Juni 1994 (1994-06-28) * Spalte 3, Absatz 5; Ansprüche 1,11; Beispiel 1 * --- | 1-6, 10-12,14 | |
| Y | WO 01 97862 A (BRAUERS GEORG ;NYCOMED AMERSHAM PLC (GB); STOREY ANTHONY EAMON (GB) 27. Dezember 2001 (2001-12-27) * Seite 8; Ansprüche 1,3,16,19 * --- | 7-9 | |
| Y | DE 101 14 352 C (EUCRO EUROP CONTRACT RES GMBH) 18. April 2002 (2002-04-18) * das ganze Dokument * ----- | 7-9,13 | |

RECHERCHIERTE SACHGEBIETE (Int.Cl.7)

Der vorliegende Recherchenbericht wurde für alle Patentansprüche erstellt

| Recherchenort | Abschlußdatum der Recherche | Prüfer |
|---|---|---|
| MÜNCHEN | 29. August 2003 | Ansaldo, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.....................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 03 01 0660

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-08-2003

| Im Recherchenbericht angeführtes Patentdokument | Datum der Veröffentlichung | Mitglied(er) der Patentfamilie | Datum der Veröffentlichung |
|---|---|---|---|
| US 6123920 A | 26-09-2000 | AU 716667 B2 | 02-03-2000 |
| | | AU 1390497 A | 01-08-1997 |
| | | CA 2242647 A1 | 17-07-1997 |
| | | CN 1212629 A | 31-03-1999 |
| | | CZ 9802194 A3 | 16-12-1998 |
| | | EA 1336 B1 | 26-02-2001 |
| | | EP 0877630 A2 | 18-11-1998 |
| | | WO 9725073 A2 | 17-07-1997 |
| | | HU 9901192 A2 | 30-08-1999 |
| | | IL 125150 A | 19-03-2001 |
| | | JP 2000504300 T | 11-04-2000 |
| | | NO 983060 A | 07-09-1998 |
| | | NZ 325403 A | 28-02-2000 |
| | | PL 327679 A1 | 21-12-1998 |
| | | US 6423296 B1 | 23-07-2002 |
| EP 0689430 A | 03-01-1996 | DE 4309333 A1 | 22-09-1994 |
| | | DE 4407338 A1 | 07-09-1995 |
| | | DE 59403734 D1 | 18-09-1997 |
| | | EP 0689430 A1 | 03-01-1996 |
| | | JP 8508721 T | 17-09-1996 |
| | | US 5916539 A | 29-06-1999 |
| | | AT 156706 T | 15-08-1997 |
| | | DE 4427821 A1 | 01-02-1996 |
| | | WO 9421240 A2 | 29-09-1994 |
| WO 9001899 A | 08-03-1990 | US 5055288 A | 08-10-1991 |
| | | AT 142891 T | 15-10-1996 |
| | | DE 68927230 D1 | 24-10-1996 |
| | | DE 68927230 T2 | 06-02-1997 |
| | | EP 0441797 A1 | 21-08-1991 |
| | | WO 9001899 A1 | 08-03-1990 |
| | | US 5314679 A | 24-05-1994 |
| WO 0189584 A | 29-11-2001 | AU 7468301 A | 03-12-2001 |
| | | EP 1283728 A2 | 19-02-2003 |
| | | WO 0189584 A2 | 29-11-2001 |
| EP 0988864 A | 29-03-2000 | EP 0988864 A1 | 29-03-2000 |
| US 4770183 A | 13-09-1988 | AT 143604 T | 15-10-1996 |
| | | CA 1301063 C | 19-05-1992 |
| | | DE 3751918 D1 | 07-11-1996 |
| | | DE 3751918 T2 | 20-03-1997 |
| | | EP 0275285 A1 | 27-07-1988 |
| | | JP 1500196 T | 26-01-1989 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 03 01 0660

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-08-2003

| Im Recherchenbericht angeführtes Patentdokument | Datum der Veröffentlichung | Mitglied(er) der Patentfamilie | Datum der Veröffentlichung |
|---|---|---|---|
| US 4770183 A | | NO 880931 A | 02-03-1988 |
| | | US 5490991 A | 13-02-1996 |
| | | US 4827945 A | 09-05-1989 |
| | | WO 8800060 A1 | 14-01-1988 |
| | | US 4951675 A | 28-08-1990 |
| | | US 5554386 A | 10-09-1996 |
| | | US 5589591 A | 31-12-1996 |
| | | US 5069216 A | 03-12-1991 |
| | | US 5679323 A | 21-10-1997 |
| | | US 5102652 A | 07-04-1992 |
| | | US 5141739 A | 25-08-1992 |
| | | US 5262176 A | 16-11-1993 |
| | | US 5284646 A | 08-02-1994 |
| | | US 5248492 A | 28-09-1993 |
| | | US 5219554 A | 15-06-1993 |
| | | US 5478576 A | 26-12-1995 |
| | | US 5352432 A | 04-10-1994 |
| | | US 5342607 A | 30-08-1994 |
| | | US 5336506 A | 09-08-1994 |
| | | US 5314679 A | 24-05-1994 |
| WO 0222011 A | 21-03-2002 | DE 10046514 A1 | 25-04-2002 |
| | | AU 1220702 A | 26-03-2002 |
| | | WO 0222011 A1 | 21-03-2002 |
| | | EP 1317208 A1 | 11-06-2003 |
| | | NO 20031204 A | 17-03-2003 |
| | | US 2002087071 A1 | 04-07-2002 |
| US 5323780 A | 28-06-1994 | AT 171784 T | 15-10-1998 |
| | | AU 4780393 A | 03-03-1994 |
| | | CA 2141534 A1 | 17-02-1994 |
| | | DE 69321359 D1 | 05-11-1998 |
| | | DE 69321359 T2 | 29-04-1999 |
| | | EP 0659056 A1 | 28-06-1995 |
| | | ES 2123663 T3 | 16-01-1999 |
| | | JP 2992582 B2 | 20-12-1999 |
| | | JP 7509716 T | 26-10-1995 |
| | | MX 9304763 A1 | 31-05-1994 |
| | | WO 9403107 A1 | 17-02-1994 |
| | | US 5462053 A | 31-10-1995 |
| WO 0197862 A | 27-12-2001 | AU 6413301 A | 02-01-2002 |
| | | CA 2411577 A1 | 27-12-2001 |
| | | CN 1437486 T | 20-08-2003 |
| | | CZ 20024112 A3 | 18-06-2003 |
| | | EP 1292338 A2 | 19-03-2003 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT
ÜBER DIE EUROPÄISCHE PATENTANMELDUNG NR.** EP 03 01 0660

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

29-08-2003

| Im Recherchenbericht angeführtes Patentdokument | Datum der Veröffentlichung | Mitglied(er) der Patentfamilie | Datum der Veröffentlichung |
|---|---|---|---|
| WO 0197862 A | | WO 0197862 A2 | 27-12-2001 |
| | | NO 20026138 A | 21-02-2003 |
| DE 10114352 C | 18-04-2002 | DE 10114352 C1 | 18-04-2002 |
| | | WO 02076515 A2 | 03-10-2002 |

EPO FORM P0461

Für nähere Einzelheiten zu diesem Anhang : siehe Amtsblatt des Europäischen Patentamts, Nr.12/82